# EUROPÄISCHE PATENTANMELDUNG

(11) **EP 1 125 592 A1**
(43) Veröffentlichungstag der Anmeldung: **22.08.2001**
(21) Anmeldenummer: 01102636.6
(22) Anmeldetag: 07.02.2001
(51) Int. Cl.: A61M 5/178, B65B 3/00

(54) **Verfahren zum Befüllen einer Spritze**

(30) Priorität: 14.02.2000 DE 10006474
(71) Anmelder: TRANSCOJECT GESELLSCHAFT FÜR MEDIZINISCHE GERÄTE mbH & CO. KG, 24539 Neumünster (DE)
(72) Erfinder: Rolle, Alexander, Dr., 24637 Schillsdorf (DE); Pipprich, Karl-Heinz, 24634 Padenstedt (DE)
(74) Vertreter: Vollmann, Heiko, Dipl.-Ing.

(57) **Zusammenfassung**

Zum Befüllen einer Spritze wird zunächst der Spritzenzylinder (1) mit einer Flüssigkeit oder einem pastösen Stoff (12) gefüllt, danach der Spritzenkolben (2) eingeführt. Über eine Öffnung im Boden des Spritzenkolbens (2) wird die im Spritzenzylinder (1) befindliche Luft abgeführt, wonach die Öffnung (11) verschlossen wird.

## Beschreibung

Die Erfindung betrifft ein Verfahren zum Befüllen einer Spritze gemäß den im Oberbegriff des Anspruchs 1 bzw. 2 angegebenen Merkmalen.

Zur Vereinfachung der Handhabung werden heutzutage vermehrt gefüllte Spritzen angeboten, sei es für medizinische Zwecke (mit Medikament gefüllte Spritze) oder auch für andere, beispielsweise technische Zwecke (die Spritze ist z. B. mit Schmierstoff oder Klebemittel gefüllt). Dies erfolgt üblicherweise dergestalt, dass der Spritzenzylinder zunächst an seinem kanülenanschlussseiten Ende durch eine Kappe dicht verschlossen wird, wonach er von der anderen Seite befüllt wird. Nachdem die gewünschte Füllstandshöhe erreicht ist, wird schließlich der Spritzenkolben in den Zylinder eingeführt. Da der Spritzenkolben gegenüber dem Spritzenzylinder abdichtet, bildet sich stets ein Luftpolster zwischen dem Kolbenboden und der Oberfläche des Füllgutes, das zu entfernen ist. Dies erfolgt üblicherweise bei den aus Kunststoff bestehenden Spritzen dadurch, dass der Spritzenzylinder an zwei entgegengesetzten Seiten kraftbeaufschlagt und soweit verformt wird, dass aufgrund des dann entstehenden ovalen Querschnitts die Luft seitlich am Kolben vorbei entweichen kann.

Dieses Entlüften seitlich am Kolben vorbei hat erhebliche Nachteile. Zum einen besteht die Gefahr, dass die Kolbendichtung durch das Verformen des Spritzenzylinders beschädigt wird. Weiterhin besteht die Gefahr, dass der Spritzenzylinder sich plastisch verformt, was zu Problemen hinsichtlich der Dichtheit zwischen Kolben und Zylinder sowie beim Gleiten des Kolbens im Zylinder führen kann. Dieses Verfahren kann darüber hinaus nicht bei den Spritzen angewandt werden, bei denen Kolben und Kolbendichtung zweiteilig, d. h. die Kolbendichtung aus einem hochelastischen Werkstoff gefertigt ist, da dann die Dichtung der Zylinderverformung folgt. Gerade diese Art von Spritzen wären jedoch zum Vorbefüllen besonders geeignet, da sie hinsichtlich Dichtheit und Handhabung beim Ausbringen des Füllstoffes erhebliche Vorteile aufweisen. Schließlich sei erwähnt, dass das vorerwähnte Befüllverfahren auch hygienische Probleme mit sich bringen kann, da es in der Regel nicht zu vermeiden ist, dass beim Entlüften ein Teil des im Zylinder befindlichen Füllguts mitgerissen wird und sich jenseits des Kolbens innerhalb des Spritzenzylinders bzw. am Kolbenschaft absetzt.

Vor diesem Hintergrund liegt der Erfindung die Aufgabe zugrunde, ein Verfahren zum Befüllen von Spritzen zu schaffen, mit dem die vorerwähnten Nachteile vermieden werden. Darüber hinaus soll eine Spritze geschaffen werden, mit der das erfindungsgemäße Verfahren durchgeführt werden kann.

Der verfahrensmäßige Teil dieser Aufgabe wird durch die in Anspruch 1 oder alternativ Anspruch 2 angegebenen Merkmale gelöst.

Der vorrichtungsmäßige Teil der Aufgabe wird durch die in Anspruch 3 bzw. Anspruch 12 angegebenen Merkmale gelöst. Vorteilhafte Ausgestaltungen der Erfindung sind in den Unteransprüchen sowie der nachfolgenden Beschreibung angegeben.

Das erfindungsgemäße Verfahren sieht statt der bisherigen Entlüftung seitlich am Kolben vorbei eine Entlüftung durch eine Öffnung im Kolbenboden vor, die nach dem Entlüften dicht verschlossen wird. Alternativ kann auch die Befüllung selbst durch diese Öffnung erfolgen, beispielsweise über ein Rohr, das vom proximalseitig durch den Kolbenschaft bis zur Öffnung geführt wird.
Das erfindungsgemäße Verfahren hat den Vorteil, dass durch den Befüll-und Entlüftungsvorgang keinerlei Belastung auf die Kolbendichtung ausgeübt wird. Es können darüber hinaus sowohl Kolben mit einstückig ausgebildeter Kolbendichtung als auch solche mit gesondert ausgebildeter Kolbendichtung, beispielsweise in Form eines elastischen O-Rings Verwendung finden. Da eine Verformung des Spritzenzylinders nicht mehr erforderlich ist, ist eine wesentlich bessere Funktionssicherheit der befüllten Spritze gegeben. Insbesondere unter Verwendung eines bis zur Öffnung reichenden Rohres kann erreicht werden, dass beim Entlüften der ggf. mitgerissene Füllstoff sich nicht mehr im Bereich des Kolbenschaftes ablagert. Dies ist insbesondere für den medizinischen Einsatz von Vorteil, da somit höchste Hygieneanforderungen erfüllt werden können.

Das erfindungsgemäße Verfahren gemäß Anspruch 1, bei dem die Entlüftung nach Einführen des Kolbens in den Spritzenzylinder erfolgt, kann weitestgehend mit vorhandenen Anlagen nach dem Stand der Technik ausgeführt werden, während das erfindungsgemäße Verfahren gemäß Anspruch 2, bei dem der Füllvorgang durch die Öffnung im Kolbenboden erfolgt, größere Modifizierungen der Anlage erfordern wird. Beiden Verfahren gemeinsam ist, dass speziell hierzu ausgebildete Spritzen zum Einsatz kommen, insbesondere mit modifizierten Spritzenkolben. Mindestanforderung hierbei ist eine Öffnung im Kolbenboden, durch die die in dem durch den Kolben abgeschlossenen Teil des Zylinderraumes befindliche Luft - bzw. das dort befindliche Gas oder Gasgemisch - abgeführt werden kann. Diese Öffnung muss nach dem Entlüftungs- bzw. Füllvorgang dicht verschlossen werden, was entweder durch einen Verschluss-stopfen, der form- und/oder kraftschlüssig dichtend in die Öffnung eingegliedert wird, oder aber in sonstiger Weise verschlossen wird. Gemäß der Erfindung kann diese Öffnung auch verschweißt, verklebt, verfüllt oder in anderer Weise verschlossen werden.

Während es bei Spritzen nach dem Stand der Technik üblich ist, an den im Wesentlichen tellerförmigen Kolben einen profilierten Schaft mit proximalseitiger Auflagefläche für den Daumen anzuformen, ist es für die erfindungsgemäße Ausbildung von Vorteil, wenn sich an den Kolbenboden ein rohrförmiger Schaft anschließt, damit die im Kolbenboden befindliche Öffnung durch den Kolbenschaft mit dem proximalen Ende des Kolbens leitungsverbunden ist. Über diesen rohrförmigen Schaft kann entlüftet bzw. befüllt werden, sei es direkt oder durch Einführen eines Rohres. Darüber hinaus kann der Verschlussstopfen durch diesen Schaft bis zur Öffnung geführt werden. Um eine Auflagefläche für den Daumen zu bilden, wie es bei Spritzenkolben proximalseitig üblich ist, wird zweckmäßigerweise am proximalen Ende des Kolbenschaftes eine flanschartige Auflage vorgesehen. Ein so gebildeter Kolben kann als einstückiges Spritzgussteil hergestellt werden. Dabei kann der Kolben wahlweise einstückig mit der Kolbendichtung ausgebildet sein oder auch eine gesonderte Kolbendichtung aus einem höher elastischem Werkstoff aufweisen.

Als Verschlusskörper für die Öffnung im Boden des Kolbens kann eine Kugel, z. B. eine Kunststoffkugel eingesetzt werden, wobei dann die Öffnung im Boden des Kolbens zweckmäßigerweise so ausgebildet ist, dass die Kugel sowohl form- als auch kraftschlüssig in der Öffnung verrastet, um einerseits einen sicheren Halt in der Öffnung zu gewährleisten und andererseits auch die Dichtwirkung zu erzeugen. Der Einsatz einer Kugel als Verschlusskörper ist montagetechnisch besonders günstig, da bei der Zufuhr der Kugel keine Orientierung zu beachten ist und im Übrigen die Separierung derartiger Kugeln technisch wenig aufwendig ist.

Von der Dichtwirkung besonders zuverlässig ist ein kegel- oder kegelstumpfförmiger Verschlusskörper, der entweder ausschließlich kraftschlüssig dichtend in die Öffnung verbracht wird oder zusätzlich auch noch verrastet werden kann. Wenn ein konischer Verschlussstopfen Verwendung findet, der orientiert in die entsprechend konisch ausgebildete Öffnung durch den Kolbenschaft eingeführt werden muss, ist es zweckmäßig, den Verschlusskörper selbst mit einem Schaft sowie mit proximalseitiger Auflage zu versehen, derart, dass beim Einschieben des Kolbens in den Zylinder die Kraftaufbringung über die verschlusskörperseitige Auflage erfolgt. Hierdurch wird erreicht, dass dann, wenn der Kolben auf die im Zylinder befindliche Flüssigkeit oder pastöse Masse Druck ausübt, zugleich auch die Kraft auf den Verschlusskörper erhöht wird, wodurch die Dichtwirkung auch beim Austragen des Stoffes durch die Spritzenspitze zuverlässig sichergestellt wird.

Vorteilhaft ist es, wenn ein solch konischer Verschlusskörper mit Schaft und proximalseitiger Auflage Verwendung findet, diesen so zu dimensionieren, dass auch die im Kolbenschaft befindliche Luft zumindest weitgehend verdrängt wird.

Die Erfindung ist nachfolgend anhand von in den Figuren dargestellten Ausführungsbeispielen näher erläutert. Es zeigen:
- Fig. 1a-d: vier Verfahrensschritte zum Befüllen einer ersten Ausführung einer erfindungsgemäßen Spritze nach dem erfindungsgemäßen Verfahren,
- Fig. 2a - d: eine alternative Ausführung einer erfindungsgemäßen Spritze in Darstellung nach Fig. 1 und
- Fig. 3: in vergrößerter Darstellung das distale Ende eines Kolbens einer weiteren Ausführung im Längsschnitt.

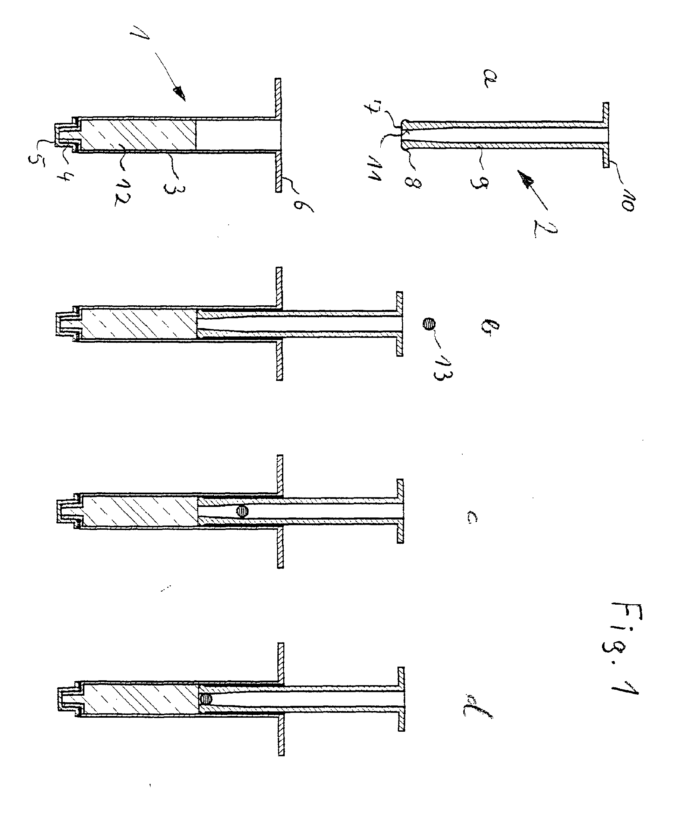

Die anhand von Fig. 1 dargestellte Spritze besteht aus einem Spritzenzylinder 1 und einem Spritzenkolben 2. Der Spritzenzylinder 1 weist einen zylindrisch rohrförmigen Teil 3 auf, der am distalen Ende durch eine stirnseitige Wand begrenzt wird, welche in einem Kanülenanschluss 4 übergeht, beispielsweise in den konusförmigen Lueranschluss oder den Luerlockanschluss mit Gewindeverriegelung. Der Kanülenanschluss 4 ist durch eine abnehmbare Verschlusskappe 5 dicht verschlossen. An das proximalseitige Ende des rohrförmigen Teils 3 schließt sich ein flanschartiger Teil 6 an, der eine Fingerauflage bildet.

Der Spritzenkolben 2 besteht aus einem, den eigentlichen Kolben bildenden Kolbenboden 7, der umfangseitig eine Kolbendichtung 8 aufweist. An den Kolbenboden 7 schließt proximalwärts ein rohrförmiger Kolbenschaft 9 an, der an seinem proximalen Ende einen nach außen gerichteten Flansch 10 aufweist.

Im Kolbenboden 7 ist eine Öffnung 11 vorgesehen, die mittig angeordnet und bündig in die Innenwandung des Kolbenschaftes 9 übergeht, wie dies in Fig. 1a deutlich zu sehen ist.

Der Spritzenkolben 2 ist so dimensioniert, dass er beim Einführen in den Spritzenzylinder 1 mit der Kolbendichtung 8 dichtend innerhalb des rohrförmigen Teils 3 anliegt und verschiebbar ist.

Zum Befüllen der Spritze mit einem flüssigen oder pastösen Stoff 12 wird der mit der Kappe 5 dicht verschlossene Spritzenzylinder 1 in der in Fig. la dargestellten Anordnung von oben, d. h. vom flanschartigen Teil 6 aus, befüllt, und zwar bis zu der gewünschten Füllstandshöhe. Nachdem dies erfolgt ist (Fig. 1a), wird der Spritzenkolben 2 in den Spritzenzylinder 1 eingeführt und so weit in diesen hineingeschoben, bis das distale Kolbenende die Oberfläche des Füllstoffs 12 erreicht hat. Dabei wird die im rohrförmigen Teil 3 zwischen dem Kolbenboden 7 und der Oberfläche des Füllstoffs 12 im rohrförmigen Teil 3 befindliche Luft über die Öffnung 11 und den Kolbenschaft 9 nach oben abgeführt (Fig. 1b). Sobald diese Stellung erreicht ist, also die zwischen Kolbenboden 7 und Füllstoff 12 befindliche Luft - dies kann auch ein sonstiges Gas oder Gasgemisch sein - entfernt ist, wird die Öffnung 11 durch einen Verschlusskörper 13 in Form einer Kugel dicht verschlossen. Hierzu wird die Kugel 13 vom flanschseitigen Ende des Spritzenkolbens 2 her in den Kolbenschaft 9 eingeführt (Fig. 1b, 1c). Da der Durchmesser der Kugel deutlich kleiner als der Innendurchmesser des rohrförmigen Kolbenschaftes 9 ist, gelangt die Kugel 13 bis nahe zum Kolbenboden 7. Die dabei verdrängte Luft kann seitlich zwischen Kolbenschaft und Kugel vorbeiströmen. Kurz vor Erreichen des Kolbenbodens 7 verjüngt sich der Kolbenschaft 9 an seiner Innenseite bis unter den Kugeldurchmesser, so dass die Kugel dort aufgrund des enger werdenden Kolbenschaftes 9 verharrt (Fig. 1c). Die Kugel 13 wird dann mittels eines vom flanschseitigen Ende in den Kolbenschaft 9 eingeführten Werkzeuges in die in Fig. 1d dargestellte Verschlusslage gepresst, in der sie formschlüssig und kraftschlüssig dichtend die Öffnung 11 verschließt. Hierzu ist innerhalb des Kolbenbodens 7 eine Art Ventilsitz für die Kugel 13 vorgesehen, der so ausgestaltet ist, dass durch Kraftschluss eine umlaufende Dichtlinie erzielt wird, wobei die Öffnung oberhalb und unterhalb dieses Dichtsitzes so verjüngt ist, dass ein Formschluss gegeben ist, der die Kugel 13 in dieser Lage hält. Beim Einpressen der Kugel in den Dichtsitz wird dieser Formschluss durch Aufweiten kurzzeitig überwunden, so dass eine Art Rastverbindung entsteht. Da dieser verengte Bereich oberhalb der Kolbendichtung 8 sitzt, wird die Kolbendichtung durch das zeitweise Aufweiten nicht beeinträchtigt, auch wenn diese, wie bei der in Fig. 1 dargestellten Ausführung einstückig mit dem übrigen Spritzenkolben 2 ausgebildet ist.

Anstelle des vorbeschriebenen Verfahrens kann der Spritzenzylinder 1 auch nach Einsetzen des Spritzenkolbens 2 befüllt werden, wobei dann das Füllen mittels eines durch den Kolbenschaft 9 bis zur Öffnung 11 durchgeführten Rohrs erfolgt, über das der Füllstoff 12 eingebracht wird. Nach Entfernen dieses Rohrs erfolgt dann das Verschließen der Öffnung 11, wie anhand der Figuren 1 b- d beschrieben.

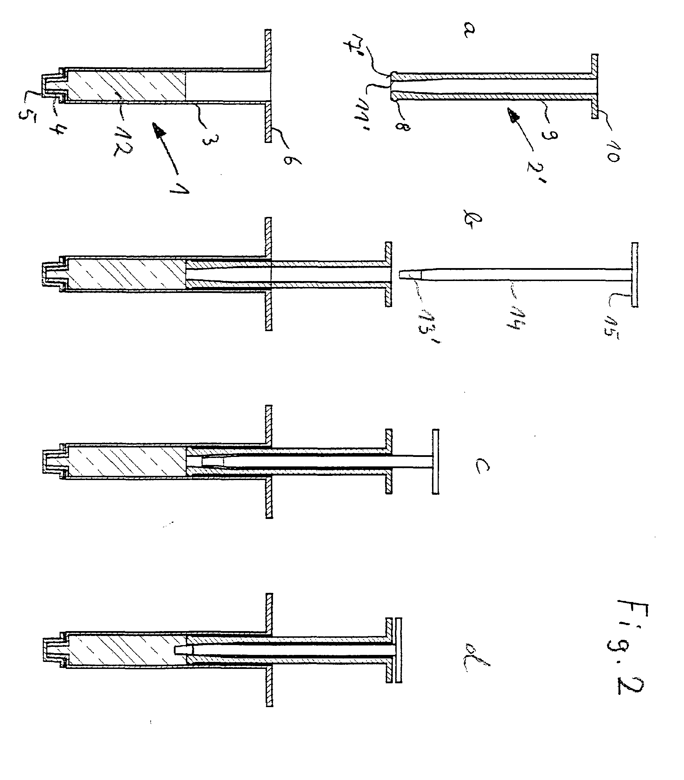

Anhand der Figuren 2a - d ist der gleiche Füllvorgang beschrieben, jedoch unterscheidet sich dort der Spritzenkolben 2 hinsichtlich der Öffnung 11 sowie des Verschlusskörpers 13.

Der in Fig. 2 dargestellte Spritzenkolben 2 ' unterscheidet sich von dem vorbeschriebenen Spritzenkolben 2 im Wesentlichen durch die Ausbildung der Öffnung 11 ' im Kolbenboden 7 '. Die Öffnung 11 ' ist zum distalen Ende hin konisch verjüngt ausgebildet. Entsprechend konisch ausgebildet ist der Verschlusskörper 13', der neben dem eigentlichen kegelstumpfförmigen Verschlusskörperteil proximalseitig einen Schaft 14 aufweist, der durch den Kolbenschaft 9 hindurchgeführt ist und diesen proximalseitig überragt. Am proximalen Ende des Schafts 14 ist eine tellerförmige Auflage 15 vorgesehen.

Wie bei der vorbeschriebenen Ausführung wird zunächst der Spritzenzylinder 1 mit Füllstoff 12 gefüllt, wonach der Spritzenkolben 2 ' eingeführt wird, bis der Kolbenboden 7 ' auf der Oberfläche des Füllstoffs 12 anliegt. Sodann wird der Verschlusskörper 13 ' vom flanschseitigen Ende des Spritzenkolbens 2 in den Kolbenschaft 9 eingeführt, wobei Verschlusskörper 13 und Schaft 14 so dimensioniert sind, dass sie mit Spiel durch den Kolbenschaft 9 geführt werden können. Seine Verschlussposition erreicht der Verschlusskörper 13 ', wenn dieser dichtend in der Öffnung 11 ' sitzt, wie dies anhand von Fig. 2d dargestellt ist. Dabei genügt grundsätzlich ein kraftschlüssiger Sitz des Verschlusskörpers 13 ' in der Öffnung 11 ', Es kann jedoch zusätzlich zwischen Schaft 14 und der Innenseite des Kolbenschaftes 9 eine Rastvorrichtung, beispielsweise durch einen Vorsprung im Kolbenschaft 9 mit entsprechender Einbuchtung im Zylinder 3 vorgesehen sein, die diese Verschlussstellung zusätzlich formschlüssig sichert. Darüber hinaus ist bei dieser Ausführung der Verschlusskörper 13 ' zusätzlich dadurch gesichert, dass im Anwendungsfall, wenn also der Spritzenkolben 2 zum Ausbringen des Füllstoffs 12 über den Kanülenanschluss 4 in den Spritzenzylinder 1 eingefahren wird, die kolbenseitige Kraftaufbringung über die Auflage 15 erfolgt, d. h. die Kraftübertragung zwingend über den Dichtsitz zwischen Verschlusskörper 13 ' und Öffnung 11 ' erfolgt.

Zur Verbesserung der Entlüftungswirkung ist es zweckmäßig, den Kolbenboden 7 ' in Richtung auf die Öffnung 11 bzw. 11 ' schräg zulaufend auszubilden, so dass die zwischen Kolbenboden 7 ' und der Oberfläche des Füllstoffs 12 befindliche Luft praktisch selbsttätig zur Öffnung hingeführt wird und eine möglichst vollständige Entlüftung erfolgen kann.

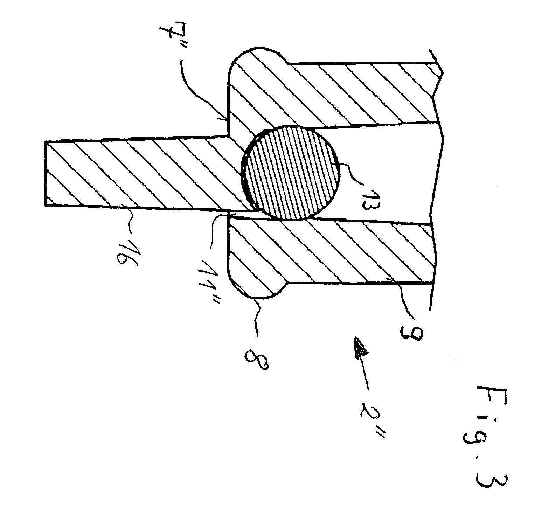

In Fig. 3 ist eine Ausführungsvariante dargestellt, bei welcher der Spritzenkolben 2 " als sogenannter Verdrängerkolben ausgebildet ist, d. h. einen distalen, in dieser Ausführung etwa zylindrischen Teil 16 aufweist, der sich vom Kolbenboden 7 " distalwärts erstreckt und dazu dient, den Spritzenzylinder 1 beim Einfahren vollständig, d. h. einschließlich des Inhalts des Kanülenanschlusses zu entleeren. Da dieser Teil 16 mittig angeordnet sein muss, ist bei dieser Ausführungsvariante die Öffnung 11 " im Kolbenboden außermittig, nämlich neben dem zylindrischen Teil 16 angeordnet. Im Übrigen ist dieser Spritzenkolben 2 " so wie der anhand von Fig. 1 beschrieben ausgebildet.

Es wird darauf hingewiesen, dass unter Kolbenboden im Sinne der Erfindung die distalseitige Stirnfläche des Spritzenkolbens zu verstehen ist. Während sich bei größervolumigen Spritzen in der Regel ein tellerartiges Bodenteil zwischen Öffnung und Kolbendichtung befindet, kann bei Spritzenkolben kleinen Durchmessers der Kolbenboden auch durch das ringförmig verdickte distale Ende des Spritzenkolbens zwischen Öffnung und Kolbendichtung gebildet sein, das stetig in den Kolbenschaft übergehen kann, wie es auch bei den vorstehend beschriebenen Ausführungsbeispielen dargestellt ist.

### Bezugszeichenliste

- 1 -: Spritzenzylinder
- 2 -: Spritzenkolben
- 2' -: Spritzenkolben in Fig. 2
- 2" -: Spritzenkolben in Fig. 3
- 3 -: rohrförmiger Teil vom Spritzenzylinder
- 4 -: Kanülenanschluss des Spritzenzylinders
- 5 -: Verschlusskappe
- 6 -: flanschartiger Teil des Spritzenzylinders
- 7 -: Kolbenboden
- 7' -: Kolbenboden in Fig. 2
- 7" -: Kolbenboden in Fig. 3
- 8 -: Kolbendichtung
- 9 -: Kolbenschaft
- 10 -: Kolbenflansch
- 11 -: Öffnung im Kolbenboden
- 11' -: Öffnung im Kolbenboden in Fig. 2
- 11 " -: Öffnung im Kolbenboden in Fig. 3
- 12 -: Füllstoff
- 13 -: Verschlusskörper
- 13' -: Verschlusskörper in Fig. 2
- 14 -: Schaft des Verschlusskörpers
- 15 -: Auflage des Verschlusskörpers
- 16 -: zylindrisches Teil in Fig. 3

## Patentansprüche

1. Verfahren zum Befüllen einer Spritze, bei dem ein an einem Ende verschlossener Spritzenzylinder (1) mit einem flüssigen oder pastösen Stoff (12) befüllt wird, wonach ein Spritzenkolben (2) in den Spritzenzylinder (1) eingeführt und in dem vom Kolben abgeschlossenen Teil des Spritzenzylinders befindliches Gas oder Gasgemisch entfernt wird, dadurch gekennzeichnet, dass das Entfernen des Gases bzw. des Gasgemisches über eine im Kolbenboden (7) vorgesehene Öffnung (11) erfolgt, die danach verschlossen wird.

2. Verfahren zum Befüllen einer Spritze mit einem flüssigen oder pastösen Stoff, dadurch gekennzeichnet, dass in einen an einem Ende verschlossenen Spritzenzylinder ein Spritzenkolben eingeführt wird, wonach der durch den Kolben abgeschlossene Teil des Spritzenzylinders durch eine Öffnung im Kolbenboden befüllt und anschließend die Öffnung verschlossen wird.

3. Spritze, insbesondere medizinische Spritze, mit einem Spritzenzylinder (1) und einem darin verschiebbar geführten Spritzenkolben (2), dadurch gekennzeichnet, dass im Kolbenboden (7) eine verschließbare Öffnung (11) vorgesehen ist.

4. Spritze nach Anspruch 3, dadurch gekennzeichnet, dass der Kolbenboden (7) einen proximalseitig daran anschließenden rohrförmigen Kolbenschaft (9) aufweist, wobei der Kolbenschaft (9) mit der Öffnung (11) im Kolbenboden (7) leitungsverbunden ist.

5. Spritze nach Anspruch 3 oder 4, dadurch gekennzeichnet, dass ein Verschlusskörper (13) vorgesehen ist, der dichtend in die Öffnung (11) einpressbar ist.

6. Spritze nach einem der vorhergehenden Ansprüche, dadurch gekennzeichnet, dass der Verschlusskörper (13 ') kegelstumpfförmig ausgebildet ist.

7. Spritze nach einem der vorhergehenden Ansprüche, dadurch gekennzeichnet, dass der Verschlusskörper (13) eine Kugel ist, die kraft-und formschlüssig in die Öffnung (11) des Kolbenbodens (7) von proximalwärts her einsetzbar ist.

8. Spritze nach einem der vorhergehenden Ansprüche, dadurch gekennzeichnet, dass an den Kolbenschaft (9) proximalseitig eine flanschförmige Auflage (10) anschließt.

9. Spritze nach einem der vorhergehenden Ansprüche, dadurch gekennzeichnet, dass das der Spritzenkolben (2) mit seinem Kolbenboden (7), dem Kolbenschaft (9) und seiner Auflage (10) einstückig als Spritzgussteil ausgebildet ist.

10. Spritze nach einem der vorhergehenden Ansprüche, dadurch gekennzeichnet, dass der Verschlusskörper (13 ') mit einem Schaft (14) mit proximalseitiger Auflage (15) versehen ist.

11. Spritze nach einem der vorhergehenden Ansprüche, dadurch gekennzeichnet, dass Verschlusskörper (13 ') und sein Schaft (14) so dimensioniert sind, dass der durch die Öffnung (11 ') und den Schaft (9) im Spritzenkolben (2) gebildete Freiraum nahezu vollständig ausfüllbar ist.

12. Kolben für eine medizinische Spritze, dadurch gekennzeichnet, dass im Kolbenboden (7) eine verschließbare Öffnung (11) vorgesehen ist.
